Europäisches Patentamt

European Patent Office

Office européen des brevets

(11) Veröffentlichungsnummer: **0 240 904 B1**

# EUROPÄISCHE PATENTSCHRIFT

(45) Veröffentlichungstag der Patentschrift: **01.07.92**

(51) Int. Cl.5: **A61K 9/20**, A61K 47/00, A61J 3/06

(21) Anmeldenummer: **87104724.7**

(22) Anmeldetag: **31.03.87**

(54) **Verfahren zur Herstellung von festen pharmazeutischen Formen.**

(30) Priorität: **11.04.86 DE 3612212**

(43) Veröffentlichungstag der Anmeldung:
**14.10.87 Patentblatt 87/42**

(45) Bekanntmachung des Hinweises auf die
Patenterteilung:
**01.07.92 Patentblatt 92/27**

(84) Benannte Vertragsstaaten:
**AT BE CH DE ES FR GB GR IT LI NL SE**

(56) Entgegenhaltungen:
EP-A- 0 104 042    EP-A- 0 121 874
EP-A- 0 125 420    EP-A- 0 204 596
EP-A- 0 214 092    DE-B- 1 229 248

PHARMAZEUTISCHE INDUSTRIE, Band 41, Nr.
4, April 1979, Editio Cantor Verlag, Aulendorf
(DE); A.A.KASSEM et al., Seiten 390-393

JOURNAL OF PHARMACEUTICAL SCIENCES,
Band 60, Nr. 9, September 1971, American
Pharmaceutical Association, Easton, PA
(US); W.L.CHIOU et al., Seiten 1281-1302

(73) Patentinhaber: **BASF Aktiengesellschaft**
**Carl-Bosch-Strasse 38**
**W-6700 Ludwigshafen(DE)**

(72) Erfinder: **Goertz, Hans-Helmut, Dr.**
**Am Wurmberg 11**
**W-6713 Freinsheim(DE)**
Erfinder: **Klimesch, Roger Guenther, Dr.**
**Georg Froeba-Str. 43**
**W-6146 Alsbach-Haehnlein 2(DE)**
Erfinder: **Laemmerhirt, Klaus, Dr.**
**Am Boehler Wald 5**
**W-6737 Boehl-Iggelheim(DE)**
Erfinder: **Lang, Siegfried, Dr.**
**Thomas Mann-Str. 22**
**W-6700 Ludwigshafen(DE)**
Erfinder: **Sanner, Axel, Dr.**
**Lorscher Ring 2 c**
**W-6710 Frankenthal(DE)**
Erfinder: **Spengler, Reinhard, Dr.**
**Comeniusstr. 6**
**W-6700 Ludwigshafen(DE)**

EP 0 240 904 B1

PHARMAZIE, Band 36, Nr. 10, Oktober 1981,
Veb VerlagVolk und Gesundheit, Berlin (DD);
R.HÜTTENRAUCH et al., Seiten 687-690

PHARMAZIE, Band 37, Nr. 3, März 1982, Veb
Verlag Volk und Gesundheit, Berlin (DD);
S.V.BOGDANOVA et al., Seiten 197-199

G. BECKMANN (1964), Promotionsarbeit, Eidgenössische Technische Hochschule in Zürich, Prom. Nr. 3531;

Cellulose-Chemie 13 (1932), S. 58-64, 71-74;

G.BECKMANN, Promotionsarbeit, 1964, Eidgenöss. Techn. Hochschule Zürich (CH),
Prom. Nr. 3531

CELLULOSE- CHEMIE, Band 13, 1932; Seiten
58-64, 71-74

**Beschreibung**

Die Erfindung betrifft ein Verfahren zur Herstellung von festen pharmazeutischen Formen, die als Bindemittel ein N-Vinylpyrrolid-2-on(NVP)-Polymerisat enthalten, durch Spritzguß oder Extrusion und Formgebung.

Die Verwendung von Polyvinylpyrrolidon in pharmazeutischen Wirkstoffmischungen, um die biologische Resorption von Wirkstoffmischungen in Form von molekularen Dispersionen zu erhöhen, ist beispielsweise aus EP-A-214 092 bekannt.

Die üblichen Tablettiermaschinen arbeiten im Taktverfahren mit Stempel und Matrize. Das Verfahren erfordert intensiv vorgemischte und speziell präparierte Tablettiermassen und ist daher insgesamt mehrstufig und aufwendig. Besonders aufwendig ist die Herstellung von festen pharmazeutischen Formen mit gezielt zeitlich dosierter Wirkstofffreigabe. Dies kann einerseits Maßnahmen zur Retardierung, andererseits Maßnahmen zur Verbesserung der Resorption von Wirkstoffen erfordern.

Eine Möglichkeit zur Verbesserung der Resorption schwerlöslicher Wirkstoffe ist die Verwendung fester Lösungen der Wirkstoffe in wasserlöslichen Polymeren. Die bisher bekannten derartigen festen Lösungen in NVP-Polymerisaten wurden über gemeinsame Lösungen von Wirkstoff und Polymer in einem organischen Lösungsmittel durch Entfernen des Lösungsmittels hergestellt. Um sowohl den hydrophoben Wirkstoff wie auch das hydrophile Polymere lösen zu können, brauchte man in der Regel chlorierte Kohlenwasserstoffe. Deren praktisch vollständige Entfernung ist sehr aufwendig. Um eine Umweltbelastung zu vermeiden, müssen die Lösungsmittel wieder möglichst vollständig aus der Abluft entfernt werden, was wiederum sehr aufwendig ist. Derartige Verfahren sind beispielsweise beschrieben in US 3 089 818; T. Tachibana und A. Nakamura, Kolloid-Zeitschrift und Zeitschrift für Polymere, Bd. 203 (1965), 130; JP-PS 24 379; M. Mayersohn et al. , J. Pharm. Sci. 55 (1966), 1323; DE-B-11 37 009; W. Scholten, Arzn. Forschung 14 ( 1964), 469.

Es wurde schon die Extrusion von Wirkstoff/polymer-Mischungen beschrieben, vgl. z. B. DE-A-12 29 248; EP-A-204 596; P. Speiser, Pharmaceutica Acta Helv. , 41 (1966), S. 340, sowie 46 (1971), S. 31. In keinem Fall wurden aber lösungsmittelfreie NVP-Polymerisate ohne Abmischung mit anderen Polymeren oder mit Wasser geschmolzen, geschweige denn extrudiert, und in keinem Fall wurde die Bildung einer festen Lösung eines in Wasser schwerlöslichen Wirkstoffs in einem wasserlöslichen Polymeren beschrieben.

In R.Voigt, Lehrbuch der pharmazeutischen Technologie, 5. Auflage, Verlag Chemie, Weinheim, 1984, Seiten 221 und 222 wird die Herstellung fester pharmazeutischer Präparate durch Spritzguß oder Extrusion und Formgebung von Wirkstoff-Thermoplast-Gemischen in allgemeiner Form beschrieben, doch ohne jegliche konkrete Angabe, insbesondere über die Art der hierfür geeigneten Polymeren. Es war hierbei zweifellos nicht an stark hydrophile Polymere, wie NVP-Polymerisate, gedacht, denn diese sind bisher im pharmazeutischen Bereich nicht über die trockene Schmelze, sondern stets mit Lösungsmittel (in der Regel Wasser) angeteigt verarbeitet worden, vgl. z.B. GB 1 388 786.

Der Erfindung lag die Aufgabe zugrunde, ein einfaches Verfahren zur Herstellung von festen pharmazeutischen Formen, vorzugsweise mit gezielt zeitlich dosierter Wirkstofffreigabe, zu entwickeln.

Die Lösung der Aufgabe besteht in einem Verfahren zur Herstellung von festen pharmazeutischen Formen durch Mischen mindestens eines pharmazeutischen Wirkstoffs mit mindestens einem schmelzbaren pharmakologisch verträglichen Bindemittel und gegebenenfalls weiteren üblichen galenischen Hilfsmitteln, bei einer Temperatur im Bereich von 50 bis 180, vorzugsweise 60 bis 160°C, Spritzgießen oder Extrudieren und Formgeben, wobei man als schmelzbares Bindemittel ein lösungsmittelfreies NVP-Polymerisat mit einem Wassergehalt von höchstens 3,5 Gew.% einsetzt, das mindestens 20, vorzugsweise mindestens 60, insbesondere 100 Gew.% NVP einpolymerisiert enthält und dessen sämtliche Comonomere, soweit vorhanden, Stickstoff und/oder Sauerstoff enthalten, und wobei zumindest dann, wenn die Glastemperatur der Mischung über 120°C liegt, ein NVP-Polymerisat eingesetzt wird, das durch Polymerisation in einem organischen Lösungsmittel oder mit einem organischen Peroxid als Starter in wäßriger Lösung erhalten wurde, und wobei die Mischung keinen in Magensaft schwerlöslichen (in 6 Stunden zu weniger als 10 % löslichen) Thermoplasten zugemischt enthält.

Die NVP-Polymerisate sollen mindestens 20, vorzugsweise mindestens 60, insbesondere 100 Gew.% NVP einpolymerisiert enthalten und einen K-Wert nach Fikentscher (Cellulose-Chemie, 13, 1932, S. 58 bis 64 und 71 bis 74) von 10 bis 70, vorzugsweise 10 bis 50, besser 12 bis 40 und insbesondere 12 bis 35, im Falle von NVP-Homopolymerisaten vorzugsweise 12 bis 35, insbesondere 12 bis 17 aufweisen.

Das polymere Bindemittel muß in der Gesamtmischung aller Komponenten im Bereich von 50 bis 180, vorzugsweise 60 bis 130°C erweichen oder schmelzen, so daß die Masse extrudierbar ist. Die Glasübergangstemperatur der Mischung muß also auf jeden Fall unter 180, vorzugsweise unter 130°C liegen.

Erforderlichenfalls wird sie durch übliche pharmakologisch akzeptable weichmachende Hilfsstoffe wie langkettige Alkohole, Ethylenglykol, Propylenglykol, Trimethylolpropan, Triethylenglykol, Butandiole, Pentanole, Hexanole, Polyethylenglykole, aromatische Carbonsäureester (z.B. Dialkylphthalate, Trimellithsäureester, Benzoesäureester, Terephthalsäureester) oder aliphatische Dicarbonsäureester (z.B. Dialkyladipate, Sebacinsäureester, Azelainsäureester, Zitronen- und Weinsäureester) oder Fettsäureester herabgesetzt. Der Weichmacher macht vorzugsweise nicht mehr als 20 Gew.% des Polymerisates aus. Besonders bevorzugt werden NVP-Polymerisate, die derartige Zusätze nicht erfordern, die also in Mischung mit dem Wirkstoff und ggf. üblichen galenischen Hilfsstoffen auch ohne speziell weichmachende Zusätze im gewünschten Temperaturbereich schmelzen oder erweichen. Das Schmelzen oder Erweichen unterhalb einer bestimmten Temperatur ist gegebenenfalls erforderlich im Hinblick auf eine mögliche thermische und/oder oxidative Schädigung nicht nur des Wirkstoffs, sondern auch des NVP-Polymerisates. Dieses könnte beim Extrudieren vergilben, weshalb die Extrusion von NVP-Polymerisaten bisher nicht üblich ist. Die Gefahr ist jedoch gering bei Extrusionstemperaturen unter 180°C, vor allem unter 130°C, wenn das Polymerisat nicht in wäßriger Lösung mit Wasserstoffperoxid als Starter hergestellt wurde, sondern in einem organischen Lösungsmittel, oder aber in Wasser mit einem organischen Peroxid als Starter, etwa nach dem Verfahren gemäß der Deutschen Patentanmeldung P 36 42 633.4 oder nach dem Verfahren von US 4 520 179 und 4 520 180.

Falls der K-Wert über 17, insbesondere über 30 oder gar 40 (bis maximal 70) liegt und keine starkweichmachende Komponente zugegen ist, kommen nur Copolymerisate mit einer Glastemperatur Tg unter 120, vorzugsweise unter 100°C in Betracht, oder das NVP-Polymerisat (einschließlich Homopolymer) darf nicht in Wasser mit $H_2O_2$ als Starter hergestellt worden sein. Dabei würden nämlich Polymer-Endgruppen entstehen, die bei höherer Temperatur zur Vergilbung führen.

Als Comonomer kommen in Betracht: ungesättigte Carbonsäuren, z.B. Methacrylsäure, Crotonsäure, Maleinsäure, Itaconsäure, sowie deren Ester mit Alkoholen mit 1 bis 12, vorzugsweise 1 bis 8 Kohlenstoffatomen, ferner Hydroxyethyl- oder Hydroxypropylacrylat und -methacrylat, (Meth)acrylamid, die Anhydride und Halbester der Maleinsäure- und Itaconsäure (wobei der Halbester vorzugsweise erst nach der Polymerisation gebildet wird), N-Vinylcaprolactam und Vinylpropionat.

Bevorzugte Comonomere sind Acrylsäure und insbesondere Vinylacetat. Es werden daher NVP-Polymerisate bevorzugt, die entweder nur NVP oder Vinylacetat als einziges Comonomeres oder doch wenigstens 10, vorzugsweise wenigstens 30 Gew.% davon einpolymerisiert enthalten. Vinylacetat und Vinylpropionat können nach der Polymerisation ganz oder teilweise verseift sein.

Mit "losungsmittelfrei" ist gemeint, daß kein organisches Lösungsmittel, insbesondere kein chlorierter Kohlenwasserstoff zugesetzt wird. Außerdem soll kein in Magensaft schwerlöslicher Thermoplast zugemischt werden, und der Wassergehalt des NVP-Polymerisates (durch spontane Aufnahme von Feuchtigkeit aus der Luft; kein absichtlicher Wasserzusatz!) soll 3,5 Gew.% nicht übersteigen. Höhere Wassergehalte sind insofern schädlich, als sie durch Verdampfung des Wassers nach dem Austritt des Polymer/Wirkstoff-Stranges aus der Düse zu porösen und unter Umständen sogar an der Oberfläche aufgeplatzten Formlingen führen.

Das erfindungsgemäße Verfahren ist beispielsweise zur Verarbeitung folgender Wirkstoffe geeignet: Betamethason, Thioctsäure, Sotalol, Salbutamol, Norfenefrin, Silymarin, Dihydergotamin, Buflomedil, Etofibrrat, Indometacin, Oxazepam, β-Acetyldigoxin, Piroxicam, Haloperidol, ISMN, Amitriptylin, Diclofenac, Nifedipin, Verapamil, Pyritinol, Nitrendipin, Doxycyclin, Bromhexin, Methylprednisolon, Clonidin, Fenofibrat, Allopurinol, Pirenzepin, Levothyroxin, Tamoxifen, Metildigoxin, o-(β-Hydroxyethyl)-rutosid, Propicillin, Aciclovirmononitrat, Paracetamol, Naftidrofuryl, Pentoxifyllin, Propafenon, Acebutolol, L-Thyroxin, Tramadol, Bromocriptin, Loperamid, Ketotifen, Fenoterol, Ca-Dobelisat, Propranolol, Minocyclin, Nicergolin, Ambroxol, Metoprolol, β-Sitosterin, Enalaprilhydrogenmaleat, Bezafibrat, ISDN, Gallopamil, Xantinolnicotinat, Digitoxin, Flunitrazepan, Bencyclan, Dexapanthenol, Pindolol, Lorazepam, Diltiazem, Piracetam, Phenoxymethylpenicillin, Furosemid, Bromazepam, Flunarizin, Erythromycin, Metoclopramid, Acemetacin, Ranitidin, Biperiden, Metamizol, Doxepin, Dikalium-Chlorazepat, Tetrazepam, Estramustinphosphat, Terbutalin, Captopril, Maprotilin, Prazosin, Atenolol, Glibenclamid, Cefaclor, Etilefrin, Cimetidin, Theophyllin, Hydromorphon, Ibuprofen, Primidon, Clobazam, Oxaceprol, Medroxyprogesteron, Flecainid, Mg-Pyridoxal-5-phosphatglutaminat, Hymechromon, Etofyllinclofibrat, Vincamin, Cinnarizin, Diazepam, Ketoprofen, Flupentixol, Molsidomin, Glibornurid, Dimetinden, Melperon, Soquinolol, Dihydrocodein, Clomethiazol, Clemastin, Glisoxepid, Kallidinogenase, Oxyfedrin, Baclofen, Carboxymethylcystein, Thioridacin, Betahistin, L-Tryptophan, Myrtol, Bromelaine, Prenylamin, Salazosulfapyridin, Astemizol, Sulpirid, Benzerazid, Dibenzepin, Acetylsalicylsäure, Miconazol, Nystatin, Ketoconazol, Na-Picosulfat, Colestyramin, Gemfibrocil, Rifampicin, Fluorcortolon, Mexiletin, Amoxicillin, Terfenadrin, Mucopolysaccharidpolyschwefelsäureester, Triazolam, Mianserin, Tiaprofensäure, Ameziniummetilsulfat, Mefloquin, Probucol, Chinidin, Carbamazepin, Mg-L-aspartat, Penbutolol, Piretanid, Amitrip-

EP 0 240 904 B1

tylin, Cyproteron, Na-Valproinat, Mebeverin, Bisacodyl, 5-Amino-Salicylsäure, Dihydralazin, Magaldrat, Phenprocoumon, Amantadin, Naproxen, Carteolol, Famotidin, Methyldopa, Auranofin, Estriol, Nadolol, Levomepromazin, Doxorubicin, Medofenoxat, Azathioprin, Flutamid, Norfloxacin, Fendilin, Prajmaliumbitartrat, Aescin.

Besonders bevorzugt werden feste Lösungen folgender Wirkstoffe: Acetaminophen (= Paracetamol), Acetohexamid, Acetyldigoxin, Acetylsalicylsäure, Acromycin, Anipamil, Benzocain, $\beta$-Carotin, Chloramphenicol, Chlordiazepoxid, Chlormadinonacetat, Chlorothiazid, Cinnarizin, Clonazepam, Codein, Dexamethason, Diazepam, Dicumarol, Digitoxin, Digoxin, Dihydroergotamin, Drotaverin, Flunitrazepam, Furosemid, Gramicidin, Griseofulvin, Hexobarbital, Hydrochlorothiazid, Hydrocortison, Hydroflumethiazid, Indomethazin, Ketoprofen, Lonetil, Medazepam, Mefrusid, Methandrostenolon, Methylprednisolon, Methylsulfadiazin (= Sulfaperin), Nalidixinsäure, Nifedipin, Nitrazepam, Nitrofurantoin, Nystatin, Östradiol, Papaverin, Phenacetin, Phenobarbital, Phenylbutazon, Phenytoin, Prednison, Reserpin, Spironolacton, Streptomycin, Sulfadimidin (= Sulfamethazin), Sulfamethizol, Sulfamethoxazol, Sulfamethoxydiazin (= Sulfameter), Sulfaperin, Sulfathiazol, Sulfisoxazol, Testosteron, Tolazamid, Tolbutamid, Trimethoprim, Tyrothricin.

Der Begriff "feste Lösungen" ist dem Fachmann geläufig, beispielsweise aus der eingangs zitierten Literatur. In festen Lösungen von pharmazeutischen Wirkstoffen in Polymeren liegt der Wirkstoff molekulardispers im Polymeren vor.

Die Bildung fester Lösungen der genannten Wirkstoffe in NVP-Polymerisaten war nicht vorherzusehen und ist umso überraschender, als viele in Wasser schwerlösliche Wirkstoffe in anderen Polymeren keine festen Lösungen (mit molekulardisperser Verteilung) bilden, sondern sich in Form fester Teilchen in das jeweilige Polymere einlagern, die elektronenmikroskopisch zu erkennen sind. Im Falle kristalliner Wirkstoffe zeigen sie auch ein Debye-Scherrer-Diagramm, im Gegensatz zu den festen Lösungen.

Falls zusätzlich zu dem erfindungsgemäß einzusetzenden noch weitere wasserlösliche, schmelzbare Bindemittel verwendet werden, soll das erstgenannte mindestens 50, vorzugsweise mindestens 70 Gew.% aller eingesetzten schmelzbaren Bindemittel ausmachen.

Feste pharmazeutische Formen im Sinne der Erfindung sind z.B. Tabletten, Drageekerne, Granulate und Zäpfchen.

Unter pharmazeutischen Wirkstoffen im Sinne der Erfindung sind alle Stoffe mit einer pharmazeutischen Wirkung und möglichst geringen Nebenwirkungen zu verstehen, sofern sie sich unter den Verarbeitungsbedingungen nicht zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, daß sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.% liegen. Auch Wirkstoff-Kombinationen können eingesetzt werden. Wirkstoffe im Sinne der Erfindung sind auch Vitamine. Mit in Wasser schwerlöslichen Wirkstoffen sind solche gemeint, deren Resorption im Magen-Darm-Trakt aufgrund ihrer zu geringen Löslichkeit normalerweise unbefriedigend ist.

Das Mischen des Wirkstoffs oder der Wirkstoffe mit den Bindemitteln und gegebenenfalls weiteren üblichen galenischen Zusätzen kann vor oder nach dem Schmelzen des polymeren Bindemittels nach den in der Technik üblichen Verfahren erfolgen. Bevorzugt wird das Mischen im Extruder mit Mischabteil, vorzugsweise einem Doppelschneckenextruder, oder im Schneckenbereich einer Spritzgußmaschine.

Die Formgebung kann über Spritzguß erfolgen oder durch Extrusion und anschließende Verformung des noch plastischen Stranges, z.B. durch Heißabschlag zu Granulat oder Verformen zu Tabletten, beispielsweise durch Hindurchführen des Stranges zwischen zwei gegenläufig angetriebenen Walzen mit einander gegenüberliegenden Vertiefungen im Walzenmantel, deren Ausführung die Tablettenform bestimmt. Auch Kaltabschlag kommt in Betracht, gegebenenfalls mit anschließendem Verpressen des Granulats zu Tabletten. Der Begriff "Extrusion" schließt - zumindest im Sinne der Erfindung - den Spritzguß mit ein.

Das NVP-Polymerisat kann über die Art und Menge an Comonomeren je nach Anwendungszweck so stark oder so schwach hydrophil eingestellt werden, daß sich die daraus hergestellten Tabletten im Mund (Buccaltablette) oder im Magen oder auch erst im Darm (rasch oder verzögert) auflösen oder so quellen, daß sie den Wirkstoff freigeben. Sie sind dann ausreichend quellbar, wenn sie bei Lagerung bei 90 % relativer Luftfeuchtigkeit mehr als 10 Gew.% Wasser aufnehmen. Falls es bei carboxylgruppenhaltigen Bindemitteln erwünscht ist, daß sie erst im alkalischen Milieu des Darms den Wirkstoff freigeben, gilt die obige Angabe der Wasseraufnahme nur für die neutralisierte Form (Salzform) des Polymeren (in der die Protonen der Carboxylgruppen ganz oder teilweise durch Ammonium-, Natrium- oder Kaliumionen ersetzt sind).

Übliche galenische Hilfsstoffe, deren Gesamtmenge bis zu 100 Gew.%, bezogen auf das Polymerisat, betragen kann, sind z.B. Streckmittel wie Silikate oder Kieselerde, Stearinsäure oder deren Salze mit z.B. Magnesium oder Kalzium, Methylcellulose, Natrium-Carboxymethylcellulose, Talkum, Saccharose, Lactose,

5

Getreide- oder Maisstärke, Kartoffelmehl, Polyvinylalkohol, ferner Netz-, Konservierungs-, Spreng-, Adsorptionsmittel, Farbstoffe, Geschmackstoffe (vgl. z.B. H. Sucker et al., Pharmazeutische Technologie, Thieme-Verlag, Stuttgart 1978).

Falls gewünscht, kann die feste pharmazeutische Form auch mit einem üblichen Überzug zur Verbesserung des Aussehens und/oder des Geschmacks (Dragee) oder zwecks zusätzlicher Verzögerung der Wirkstofffreigabe versehen werden. Für oral einzunehmende Tabletten mit verzögerter Wirkstofffreisetzung kann es günstig sein, wenn man die Tablette nach einer der bekannten Techniken in geschlossenzellig poröser Form herstellt, damit sie im Magen aufschwimmt und dadurch länger dort verweilt.

Das erfindungsgemäße Verfahren erlaubt bei festen pharmazeutischen Formen mit rascher Wirkstofffreisetzung eine wesentlich freiere Gestaltung der pharmazeutischen Form als die herkömmliche Tablettenpreßtechnik. Beispielsweise können Gravuren zur Kennzeichnung angebracht werden, oder es können fast beliebige Formen hergestellt werden, die auch für Sehbehinderte eine eindeutige Kennzeichnung darstellen. Bestimmte Formen, z.B. die Halbkugel, können auch zur Erzielung einer bestimmten Wirkstofffreisetzungscharakteristik geeignet sein. Durch Extrusion und Heiß- oder Kaltabschlag des Stranges können sehr kleinteilige und gleichmäßig geformte Granulate in einfacher Weise hergestellt werden, beispielsweise für multiple-unit-Formen.

Die in den Beispielen genannten Teile und Prozente beziehen sich auf das Gewicht. Die Wirkstoff-Freisetzungszeit wurde nach der half-change-test-Methode bestimmt.

Beispiel 1

45 Teile eines Copolymerisats vom K-Wert 30 aus 60 Gew.% N-Vinylpyrrolidon und 40 Gew.% Vinylacetat, 5 Teile Stearylalkohol und 50 Teile Theophyllin wurden in einer Spritzgußmaschine zu Drageekernen verarbeitet. Die Verarbeitungstemperatur betrug 100 °C. Die hierbei erhaltenen Drageekerne waren gegen mechanische Einflüsse stabil und zeigten keinen Abrieb beim Transport und Verpacken. Der Wirkstoff wurde beim half-change-Test, vgl. z.B. R. Voigt, Lehrbuch der pharmazeut. Technologie, 5. Aufl., Verl. Chemie, Weinheim; Deerfield Beach, Florida; Basel, 1984, S. 627, in Verbindung mit der Paddle-Methode nach USP 21 innerhalb 6 bis 8 Stunden vollständig freigesetzt.

Beispiel 2

50 Teile des Copolymerisats von Beispiel 1 und 50 Teile Theophyllin wurden in einer Spritzgußmaschine zu Oblongtabletten von 1 cm Länge verarbeitet. Die Verarbeitungstemperatur betrug 120 °C. Auch die so erhaltenen Tabletten waren gegen mechanische Einflüsse stabil und gaben den Wirkstoff innerhalb 1 bis 2 Stunden vollständig frei.

Beispiel 3

47,5 Teile eines Copolymerisats vom K-Wert 30 aus 60 Gew.% N-Vinylpyrrolidon und 40 Gew.% Vinylacetat, 2,5 Teile vernetztes Polyvinylpyrrolidon (PVP) als Tablettensprengmittel und 50 Teile Theophyllin wurden in einem Doppelschneckenextruder vermischt und extrudiert. Die Temperatur der fünf Schüsse betrug jeweils 120 °C. Die Düse hatte eine Temperatur von 130 °C. Der noch plastische Strang wurde mit Hilfe der in der deutschen Parallel-Anmeldung P 36 12 211.4 beschriebenen Vorrichtung zu Oblongtabletten verpreßt. Die Tabletten waren stabil gegen mechanische Einflüsse. Die Wirkstoff-Freisetzungszeit betrug 30 bis 45 min.

Beispiel 4

50 Teile eines Copolymerisats vom K-Wert 52 aus 30 Gew.% N-Vinylpyrrolidon und 70 Gew.% Vinylacetat und 50 Teile Theophyllin wurden in einem Doppelschneckenextruder gemischt und extrudiert. Die Temperatur der fünf Schüsse betrug 30, 60, 100, 100 und 120 °C. Die Düse wurde ebenfalls auf 120 °C aufgeheizt. Der noch plastische Strang wurde wie in Beispiel 3 zu mechanische stabilen Oblongtabletten verpreßt. Der Wirkstoff wurde innerhalb 8 Stunden vollständig freigesetzt.

Beispiel 5

47,5 Teile eines Copolymerisats vom K-Wert 30 aus 60 Gew.% N-Vinylpyrrolidon und 40 Gew.% Vinylacetat, 2,5 Teile Stearylalkohol und 50 Teile Theophyllin wurden in einer Spritzgußmaschine bei 100 °C aufgeschmolzen und zu Drageekernen verarbeitet. Die Form wurde bei Raumtemperatur belassen. Die so hergestellten Drageekerne waren gegen mechanische Einflüsse stabil. Der Wirkstoff wurde innerhalb 6 Stunden vollständig freigesetzt.

Für die Beispiele 6 bis 11 wurde jeweils eine Mischung von 50 Gew.% eines NVP-Homopolymeren (PVP) vom K-Wert nach Fikentscher 12 bis 60 und 50 Gew.% Theophyllin in einem Einwellenextruder bei folgenden Temperaturen verarbeitet:

| Beispiel | K-Wert | T [$^0$C] | | | | | Düse |
|---|---|---|---|---|---|---|---|
| | | 1. | 2. | 3. | 4. | 5. | |
| | | | | Schuß | | | |
| 6 | 12 | 115 | 125 | 135 | 135 | 135 | 145 |
| 7 | 17 | 125 | 125 | 135 | 145 | 145 | 155 |
| 8 | 25 | 145 | 155 | 165 | 175 | 175 | 175 |
| 9 | 30 | 150 | 160 | 160 | 170 | 180 | 180 |
| 10 | 60 | 150 | 160 | 160 | 170 | 180 | 180 |
| 11 | 60 | 80 | 100 | 130 | 140 | 150 | 160 |

Aus den so erhaltenen Tabletten löste sich der Wirkstoff (im künstlichen Magensaft) bei den Beispielen 6 und 7 innerhalb weniger als 30 min. vollständig, bei den Beispielen 8 und 9 innert 1 bis 2 Stunden, bei Beispiel 10 nach mehr als 2 Stunden. Bei Beispiel 11 enthielt das PVP 10 Gew.% Stearylalkohol. In diesem Fall betrug die Freisetzungszeit 8 Stunden.

Beispiele 12 bis 14

36 Teile eines Copolymerisates vom K-Wert 30 aus 60 Gew.% N-Vinylpyrrolidon und 40 Gew.% Vinylacetat, 4 Teile Stearylalkohol, 40 Teile Theophyllin und 20 Teile
Beispiel 12: Stärke
Beispiel 13: Lactose
Beispiel 14: Saccharose
wurden in einem 6-schüssigen Doppelschneckenextruder gemischt und analog Beispiel 1 zu Tabletten verformt. Die Temperatur der Schüsse betrug 90, 100, 110, 120, 130, 130°C, die der Düse 135°C. Aus den Tabletten löste sich der Wirkstoff innerhalb 6 Stunden vollständig.

Beispiel 15

50 Teile des Copolymerisates der Beispiele 12 bis 14 und 50 Teile Lithiumcarbonat wurden auf dem gleichen Gerät und bei den gleichen Temperaturen wie bei den Beispielen 12 bis 14 zu Tabletten verarbeitet, die den Wirkstoff (in künstlichem Magensaft) innerhalb 15 bis 20 min vollständig freisetzten.

Beispiel 16

50 Teile des Copolymerisates der Beispiele 12 bis 14 und 50 Teile Verapamil wurden gemäß den Beispielen 12 bis 14 zu Tabletten geformt. Die Wirkstoff-Freisetzungszeit lag hier bei etwa 3 Stunden.

Die zur Herstellung fester Lösungen eingesetzten Copolymerisate hatten folgende Zusammensetzungen und K-Werte:
A) 60 Gew.% NVP und 40 % Vinylacetat; K-Wert ca. 33.
B) 100 Gew.% NVP; K-Wert 30.
C) 100 Gew.% NVP; K-Wert 12.
D) 100 Gew.% NVP; K-Wert 17.
Die Polymeren B, C und D wurden gemäß der Deutschen Patentanmeldung P 36 42 633.4 in Wasser mit einem organischen Peroxid als Starter hergestellt.

Beispiel 17

7

3 Teile des Copolymerisates A und 1,5 Teile Benzocain wurden in einem Pflugscharmischer vorgemischt und in einem einfachen, 6schüssigen Extruder bei folgenden Temperaturen in den einzelnen Schüssen, gezählt in Richtung zur Düse hin, extrudiert: 30, 30, 40, 50, 60, 70°C. Die Düsentemperatur betrug ebenfalls 70°C. Das Extrudat bestand aus einer festen Lösung, wie die Debye-Scherrer-Aufnahme, die nicht den geringsten Hinweis auf Kristallinität enthielt, zeigte. In analoger Weise wurde - mit gleichem Ergebnis - bei den übrigen Beispielen (Tabelle) verfahren.

| Beispiel Nr. | Wirkstoff | Polymer | Wirkstoff/Polymer Gew.-Verhältnis | T1 | T2 | T3 | T4 | T5 | T6 | Düsen-Temp. °C |
|---|---|---|---|---|---|---|---|---|---|---|
| 17a | Benzocain | A | 1:3 | 20 | 30 | 40 | 40 | 50 | 50 | 50 |
| 18 | Paracetamol | C | 1:3 | 60 | 80 | 100 | 120 | 120 | 120 | 120 |
| 19 | Phenytoin | C | 1:3 | 60 | 80 | 100 | 120 | 120 | 120 | 120 |
| 20 | Benzocain | C | 1:3 | 50 | 50 | 60 | 60 | 70 | 80 | 90 |
| 21 | Indomethacin | A | 1:3 | 50 | 60 | 70 | 80 | 80 | 80 | 80 |
| 22 | Indomethacin | A | 1:3 | 60 | 80 | 100 | 120 | 120 | 120 | 120 |
| 23 | Anipamil | A | 1:3 | 30 | 30 | 40 | 50 | 50 | 60 | 60 |
| 24 | Anipamil | C | 1:1 | 30 | 30 | 40 | 50 | 50 | 60 | 60 |
| 25 | Benzocain | A | 1:1 | 30 | 30 | 40 | 500 | 50 | 60 | 60 |
| 26 | Benzocain | B | 1:1 | 30 | 30 | 40 | 500 | 50 | 60 | 60 |
| 27 | Benzocain | C | 1:1 | 30 | 30 | 40 | 50 | 50 | 60 | 60 |
| 28 | Phenytoin | A | 1:3 | 60 | 80 | 100 | 120 | 120 | 120 | 130 |
| 29 | Paracetamol | A | 1:3 | 60 | 80 | 100 | 120 | 120 | 120 | 130 |
| 30 | Sulfathiazol | A | 1:1 | 70 | 90 | 100 | 100 | 100 | 100 | 130 |
| 31 | Benzocain | A | 1:3 | 50 | 50 | 60 | 60 | 70 | 80 | 90 |
| 32 | Benzocain | C | 1:3 | 50 | 50 | 60 | 60 | 70 | 80 | 80 |
| 33 | Sulfathiazol | C | 1:3 | 70 | 90 | 100 | 100 | 100 | 100 | 120 |
| 34 | Benzocain | A | 1:9 | 30 | 40 | 50 | 60 | 60 | 70 | 70 |
| 35 | Benzocain | A | 1:6 | 50 | 60 | 70 | 80 | 85 | 85 | 85 |
| 36 | Benzocain | C | 1:9 | 30 | 30 | 40 | 50 | 60 | 60 | 60 |
| 37 | Benzocain | C | 1:6 | 30 | 40 | 60 | 60 | 70 | 70 | 70 |
| 38 | Benzocain | B | 1:9 | 60 | 70 | 90 | 90 | 90 | 100 | 100 |
| 39 | Benzocain | B | 1:6 | 60 | 80 | 80 | 100 | 120 | 120 | 120 |
| 40 | Benzocain | B | 1:3 | 60 | 80 | 80 | 100 | 120 | 120 | 120 |
| 41 | Benzocain | B | 1:1 | 60 | 80 | 95 | 100 | 120 | 135 | 140 |
| 42 | Phenytoin | A | 1:9 | 60 | 70 | 90 | 90 | 90 | 100 | 100 |

| Beispiel Nr. | Wirkstoff | Polymer | Wirkstoff/Polymer Gew.-Verhältnis | T1 | T2 | T3 | T4 | T5 | T6 | Düsen-Temp. °C |
|---|---|---|---|---|---|---|---|---|---|---|
| 43 | Phenytoin | A | 1:6 | 60 | 70 | 90 | 100 | 100 | 100 | 100 |
| 44 | Phenytoin | C | 1:9 | 60 | 70 | 90 | 90 | 90 | 100 | 100 |
| 45 | Phenytoin | C | 1:6 | 60 | 70 | 90 | 100 | 100 | 100 | 100 |
| 46 | Phenytoin | B | 1:9 | 60 | 80 | 110 | 130 | 150 | 150 | 150 |
| 47 | Phenytoin | B | 1:6 | 60 | 80 | 110 | 130 | 150 | 160 | 160 |
| 48 | Phenytoin | B | 1:3 | 60 | 80 | 110 | 120 | 150 | 160 | 170 |
| 49 | Phenytoin | B | 1:1 | 60 | 80 | 125 | 150 | 160 | 175 | 180 |

T1 bis T6 sind die Temperaturen der Schüsse 1 bis 6 in °C.

Beispiel 50

Die Beispiele 18, 19 und 20 wurden auf einer Spritzgußmaschine mit der Düsentemperatur 130°C wiederholt. Man erhielt aus festen Lösungen bestehende Tabletten.

Beispiel 51

Vitamin C als Wirkstoff wurde im Gewichtsverhältnis 1:1 mit folgenden NVP-Polymerisaten in einem Doppelschneckenextruder vermischt und bei den in der Tabelle angegebenen Temperaturen in den Schüssen 1 bis 6 und der Düse extrudiert und gemäß der deutschen Parallelanmeldung P 36 12 211.4 über einen Kalander zu Tabletten geformt.

    a) Copolymerisat aus 60 Gew.% NVP und 40 Gew.% Vinylacetat; K-Wert ca. 33.

    b) 90 Gew.% Copolymerisat gem. (a) und 10 Gew.% Stearylalkohol.

    c) Homopolymerisat aus NVP; K-Wert 17.

|  | T1 | T2 | T3 | T4 | T5 | T6 | Düse |
|---|---|---|---|---|---|---|---|
| a) | 60 | 80 | 100 | 110 | 120 | 120 | 120 °C |
| b) | 60 | 80 | 80 | 100 | 100 | 110 | 110 °C |
| c) | 60 | 80 | 100 | 110 | 120 | 120 | 125° C |

Das Vitamin wurde in allen drei Fällen in Wasser innerhalb 1 bis 2 Stunden freigesetzt. Es blieb bei der beschriebenen Verarbeitung 100%ig erhalten und war in dieser Form gegen Einwirkungen von Licht und Luftsauerstoff bei längerer Lagerung geschützt.

**Patentansprüche**

1. Verfahren zur Herstellung von festen pharmazeutischen Formen durch Mischen mindestens eines pharmazeutischen Wirkstoffs mit mindestens einem schmelzbaren pharmakologisch verträglichen Bindemittel und gegebenenfalls weiteren üblichen galenischen Hilfsmitteln und Spritzgießen oder Extrudieren und Formgeben bei einer Temperatur im Bereich von 50 bis 180°C, dadurch gekennzeichnet, daß man als schmelzbares Bindemittel ein lösungsmittelfreies N-Vinylpyrrolidon-Polymerisat mit einem Wassergehalt von höchstens 3,5 Gew.-% einsetzt, das mindestens 20 Gew.-% N-Vinylpyrrolidon-2- (NVP) einpolymerisiert enthält, wobei sämtliche gegebenenfalls einpolymerisierten Comonomeren Stickstoff und/oder Sauerstoff enthalten, und daß zumindest dann, wenn die Glastemperatur der Mischung über 120°C liegt, ein NVP-Polymerisat eingesetzt wird, das durch Polymerisation in einem organischen Lösungsmittel oder mit einem organischen Peroxid als Starter in wäßriger Lösung erhalten wurde, und daß die Mischung keinen in Magensaft schwerlöslichen (in 6 Stunden zu weniger als 10 % löslichen) Thermoplasten zugemischt enthält.

2. Verfahren nach Anspruch 1, dadurch gekennzeichnet, daß man ein polymeres Bindemittel einsetzt, das mindestens 60 Gew.% NVP einpolymerisiert enthält.

3. Verfahren nach einem der Ansprüche 1 oder 2, dadurch gekennzeichnet, daß man höchstens 20 Gew.%, bezogen auf das Polymerisat, an Weichmachern einsetzt.

4. Verfahren nach einem der Ansprüche 1 bis 3, dadurch gekennzeichnet, daß man ein polymeres Bindemittel einsetzt, das aus Polyvinylpyrrolidon besteht oder neben N-Vinylpyrrolidon nur Vinylacetat einpolymerisiert enthält.

5. Verfahren nach einem der Ansprüche 1 bis 3, dadurch gekennzeichnet, daß man ein polymeres Bindemittel einsetzt, dessen Comonomere aus folgender Gruppe ausgewählt sind: Acrylsäure, Methacrylsäure, Crotonsäure, Maleinsäure(anhydrid), Itaconsäure(anhydrid) oder Ester der genannten Säuren oder Halbester der genannten Dicarbonsäuren mit Alkoholen mit 1 bis 12 Kohlenstoffatomen, oder Hydroxyethyl- oder Hydroxypropylacrylat oder -methacrylat, Acrylamid, Methacrylamid, N-Vinylcaprolactam und Vinylpropionat.

6. Verfahren nach einem der Ansprüche 1 bis 5, dadurch gekennzeichnet, daß man einen in Wasser schwerlöslichen Wirkstoff einsetzt, der sich in der Polymerschmelze ohne Zusatz von Lösungsmitteln oder Wasser molekulardispers löst und nach dem Erstarren der Schmelze eine feste Lösung bildet.

**7.** Verfahren nach Anspruch 6, dadurch gekennzeichnet, daß man dazu mindestens einen Wirkstoff aus folgender Gruppe einsetzt: Acetaminophen (= Paracetamol), Acetohexamid, Acetyldigoxin, Acetylsalicylsäure, Acromycin, Anipamil, Benzocain, β-Carotin, Chloramphenicol, Chlordiazepoxid, Chlormadinonacetat, Chlorothiazid, Cinnarizin, Clonazepam, Codein, Dexamethason, Diazepam, Dicumarol, Digitoxin, Digoxin, Dihydroergotamin, Drotaverin, Flunitrazepam, Furosemid, Gramicidin, Griseofulvin, Hexobarbital, Hydrochlorothiazid, Hydrocortison, Hydroflumethiazid, Indomethazin, Ketroprofen, Lonetil, Medazepam, Mefrusid, Methandrostenolon, Methylprednisolon, Methylsulfadiazin (= Sulfaperin), Nalidixinsäure, Nifedipin, Nitrazepam, Nitrofurantoin, Nystatin, Östradiol, Papaverin, Phenacetin, Phenobarbital, Phenylbutazon, Phenytoin, Prednison, Reserpin, Spironolacton, Streptomycin, Sulfadimidin (= Sulfamethazin), Sulfamethizol, Sulfamethoxazol, Sulfamethoxydiazin (= Sulfameter), Sulfaperin, Sulfathiazol, Sulfisoxazol, Testosteron, Tolazamid, Tolbutamid, Trimethoprim, Tyrothricin.

**8.** Verfahren nach einem der Ansprüche 1 bis 7, dadurch gekennzeichnet, daß man ein NVP-Polymerisat vom K-Wert nach Fikentscher im Bereich von 10 bis 50 einsetzt.

**9.** Verfahren nach einem der Ansprüche 1 bis 7, dadurch gekennzeichnet, daß man ein NVP-Polymerisat vom K-Wert nach Fikentscher im Bereich von 12 bis 35 einsetzt.

**Claims**

**1.** A process for the preparation of a solid pharmaceutical form by mixing one or more pharmaceutical active compounds with one or more fusible pharmacologically tolerated binders and, if required, other conventional pharmaceutical auxiliaries, and subjecting the mixture to injection molding or extrusion and shaping at from 50 to 180°C, wherein the fusible binder used is a solvent-free N-vinylpyrrolidone polymer which has a water content of not more than 3.5% by weight and contains not less than 20% by weight of N-vinylpyrrolid-2-one (NVP) as copolymerized units, all copolymerized comonomers which may be present containing nitrogen and/or oxygen and, at least when the glass transition temperature of the mixture is above 120°C, an NVP polymer is used which is obtained by polymerization in an organic solvent or using an organic peroxide as an initiator in aqueous solution, and the mixture does not contain any thermoplastics which are sparingly soluble in gastric juice (soluble to an extent of less than 10% over 6 hours).

**2.** A process as claimed in claim 1, wherein the polymeric binder used contains not less than 60% by weight of NVP as copolymerized units.

**3.** A process as claimed in claim 1 or 2, wherein plasticizers are used in an amount of not more than 20% by weight, based on the polymer.

**4.** A process as claimed in any of claims 1 to 3, wherein the polymeric binder used consists of polyvinylpyrrolidone or contains, in addition to N-vinylpyrrolidone, only vinyl acetate as copolymerized units.

**5.** A process as claimed in any of claims 1 to 3, wherein a polymeric binder is used whose comonomers are selected from the following group: acrylic acid, methacrylic acid, crotonic acid, maleic acid, maleic anhydride, itaconic acid, itaconic anhydride and esters of the stated acids or half esters of the stated dicarboxylic acids with alcohols of 1 to 12 carbon atoms, and hydroxyethyl and hydroxypropyl acrylate and methacrylate, acrylamide, methacrylamide, N-vinylcaprolactam and vinyl propionate.

**6.** A process as claimed in any of claims 1 to 5, wherein the active compound used is sparingly soluble in water, dissolves in the polymer melt without the addition of a solvent or water to give a molecular disperse solution, and forms a solid solution after the melt solidifies.

**7.** A process as claimed in claim 6, wherein one or more active compounds from the following group are used: acetaminophen (paracetamol), acetohexamide, acetyldigoxin, acetylsalicylic acid, acromycin, anipamil, benzocaine, ß-carotene,chloramphenicol, chlordiazepoxide, chlormadinone acetate, chlorothiazide, cinnarizine, clonazepam, codeine, dexamethasone, diazepam, dicoumarol, digitoxin, digoxin, dihydroergotamine, drotaverine, flunitrazepam, furosemide, gramicidin, griseofulvin, hexobarbital, hydrochlorothiazide, hydrocortisone, hydrofluormethiazide, indomethacin, ketoprofen, lonetol, medazepam, mefruside, methandrostenolone, methylprednisolone, methylsulfadiazine (sulfaperin), nalidixic acid, nifedipine, nitrazepam, nitrofurantoin, nystatin, estradiol, papaverine, phenacetin, phenobarbital, phenylbutazone, phenytoin, prednisone, reserpine, spironolactone, streptomycin, sulfadimidine (sulfamethazine), sulfamethizole, sulfamethoxazole, sulfamethoxydiazine (sulfameter), sulfaperin, sulfathiazole, sulfisoxazole, testosterone, tolazamide, tolbutamide, trimethoprim, tyrothricin.

8. A process as claimed in any of claims 1 to 7, wherein an NVP polymer having a Fikentscher K value of from 10 to 50 is used.

9. A process as claimed in any of claims 1 to 7, wherein an NVP polymer having a Fikentscher K value of from 12 to 35 is used.

**Revendications**

1. Procédé de préparation de formes pharmaceutiques solides par mélange d'au moins une substance active pharmaceutique avec au moins un liant fusible acceptable pour l'usage pharmaceutique et la cas échéant d'autres produits auxiliaires galéniques usuels et moulage par injection ou extrusion et formage à une température dans l'intervalle de 50 à 180 degrés C, caractérisé en ce que l'on utilise en tant que liant fusible un polymère de la N-vinylpyrrolidone exempt de solvant d'une teneur en humidité de 3,5 % en poids au maximum, contenant à l'état polymérisé au moins 20 % en poids de N-vinylpyrrolidone-2 (NVP), tous les comonomères éventuellement copolymérisés contenant de l'azote et/ou de l'oxygène, et en ce que, au moins lorsque la température de transition vitreuse du mélange est supérieure à 120 degrés C, on utilise un polymère de la NVP qui a été obtenu par polymérisation dans un solvant organique ou à l'aide d'un inducteur consistant en un peroxyde organique en solution aqueuse, et en ce que le mélange ne contient pas de résine thermoplastique peu soluble dans le suc gastrique (soluble à moins de 10 % en 6 h).

2. Procédé selon la revendication 1, caractérisé en ce que l'on utilise un liant polymère contenant au moins 60 % en poids de NVP à l'état polymérisé.

3. Procédé selon la l'une des revendications 1 ou 2, caractérisé en ce que l'on utilise au maximum 20 % en poids, par rapport au polymère, de plastifiants.

4. Procédé selon l'une des revendications 1 à 3, caractérisé en ce que l'on utilise un liant polymère consistant en polyvinylpyrrolidone ou qui contient en plus de la N-vinylpyrrolidone, à l'état polymérisé, uniquement de l'acétate de vinyle.

5. Procédé selon l'une des revendications 1 à 3, caractérisé en ce que l'on utilise un liant polymère dont les comonomères sont choisis dans le groupe suivant :
l'acide acrylique, l'acide méthacrylique, l'acide crotonique, l'acide (anhydride) maléique, l'acide (anhydride) itaconique ou les esters des acides en question ou les hémiesters des acides dicarboxyliques en question et d'alcools en C 1-C 12, ou l'acrylate ou le méthacrylate d'hydroxyéthyle ou d'hydroxypropyle, l'acrylamide, le méthacrylamide, le N-vinylcaprolactame et le propionate de vinyle.

6. Procédé selon l'une des revendications 1 à 5, caractérisé en ce que l'on utilise une substance active peu soluble dans l'eau, qui se dissout à l'état de dispersion moléculaire dans le polymère fondu sans adjonction de solvant ni d'eau et qui forme après solidification de la masse fondue une solution solide.

7. Procédé selon la revendication 6, caractérisé en ce que l'on utilise à cet effet au moins une substance active du groupe suivant :
Acétaminophène (= Paracétamol), Acétohexamide, Acetyldigoxine, Acide acetylsalicylique, Acromycine, Anipamile, Benzocaine, -Carotine, Chloramphénicole, Chlordiazepoxide, Chlormadinonacétate, Chlorothiazide, Cinnarizine, Clonazepame, Codéine, Dexamethasone, Diazepame, Dicumarole, Digitoxine, Digoxine, Dihydroergotamine, Drotavérine, Flunitrazépame, Furosémide, Gramicidine, Griséofulvine, Hexobarbital, Hydrochlorothiazide, Hydrocortisone, Hydrofluméthiazide, Indométhazine, Ketoprofène,Lonétil, Medazépame, Méfruside, Méthandrosténolone, Méthylprednisolone, Méthylsulfadiazine (= Sulfapérine), Nalidixinsaure, Nifédipine, Nitrazépame, Nitrofurantoine, Nystatine, Ostradiole, Papavérine, Phenacétine, Phénobarbitale, Phénylbutazone, Phénytoine, Prednison, Réserpine, Spironolactone, Streptomycine, Sulfadimidine (= Sulfaméthazine), Sulfaméthizol, Sulfaméthoxazole, Sulfaméthoxydiazine (= Sulfaméter), Sulfapéine, Sulfathizole, Sulfisoxazole, Testostérone, Tolazamide, Tolbutamide, Triméthoprime, Tyrothricine.

8. Procédé selon l'une des revendications 1 à 7, caractérisé en ce que l'on utilise un polymère de la NVP présentant un indice K selon Fikentscher dans l'intervalle de 10 à 50.

9. Procédé selon l'une des revendications 1 à 7, caractérisé en ce que l'on utilise un polymère de la NVP ayant un indice K selon Fikentscher dans l'intervalle de 12 à 35.